# EUROPEAN PATENT APPLICATION

(11) **EP 4 732 752 A1**
(43) Date of publication of application: **29.04.2026**
(21) Application number: 25207449.7
(22) Date of filing: 08.10.2025
(51) Int. Cl.: A61B 3/00, A61B 3/032, A61B 3/12

(54) **MODEL EYE FOR EXAMINING RESOLUTION OF FUNDUS EXAMINING DEVICE**

(30) Priority: 22.10.2024 KR 20240144815
(71) Applicant: Huvitz Co., Ltd., Anyang-si, Gyeonggi-do 14055 (KR)
(72) Inventor: JO, Sun Hyung, 14055 Anyang-si (KR); BEAK, Woo Kyung, 14055 Anyang-si (KR)
(74) Representative: Henkel & Partner mbB

(57) **Abstract**

A model eye for examining the resolution of a fundus examining device is disclosed. The model eye (10) comprises: one or more lenses (100, 200) corresponding to a cornea and/or a crystalline lens of an eye; and a chart unit (300) located at an inner rear surface of the model eye (10) corresponding to a fundus of an eye, and including two or more chart plates (310, 320, 330) each having one or more resolution test chart (410, 420, 430) for examining resolution of a fundus examining device, wherein the chart plates (310, 320, 330) are arranged at different positions so that the resolution test charts (410, 420, 430) are located at the focal distances of the measurement lights of the fundus examining device which incident to a central region and peripheral region of the inner rear surface of the model eye (10) through the lenses (100, 200).

## Description

### CROSS-REFERENCE TO RELATED APPLICATION

This application claims benefit of priority to Korean Patent Application No. 10-2024-0144815filed on October 22, 2024, the entire contents of which are incorporated herein by reference.

### FIELD OF THE DISCLOSURE

The present invention relates to a model eye, and more particularly, to a model eye for examining the resolution of a fundus examining device.

### BACKGROUND

In general, a fundus examining device irradiates measurement light onto the retina of a subject eye and detects signal light reflected from the retina to examine the condition of the retina. Alternatively, a fundus examining device illuminates a retina with illumination light and obtains a retinal image by taking a picture of the retina illuminated by the illumination light using a camera. The fundus examining device includes a scanning laser ophthalmoscopy (SLO), a fundus camera and so on.

To evaluate the performance of such fundus examining device or to calibrate the fundus examining device, a standard, i.e., a model eye, that simulates a subject eye having a concave retina, is required. However, until now, a model eye specifically designed to examine the resolution (resolving power) of a fundus examining device is not known.

### [Prior Art Documents]

(Patent Document 1) Korean Patent No. 10-2475140 (December 2, 2022)
(Patent Document 2) Korean Patent No. 10-2394032 (April 29, 2022)

### SUMMARY OF THE DISCLOSURE

### TECHNICAL OBJECTS

An object of the present invention is to provide a model eye capable of examining the resolution of a fundus examining device.

Another object of the present invention is to provide a model eye capable of examining both a resolution for central region and a resolution for peripheral region of a retina of a fundus examining device.

### TECHNICAL SOLUTION

To achieve the above objects, the present invention provides a model eye (10) comprising: one or more lenses (100, 200) corresponding to a cornea and/or a crystalline lens of an eye; and a chart unit (300) located at an inner rear surface of the model eye (10) corresponding to a fundus of an eye, and including two or more chart plates (310, 320, 330) each having one or more resolution test chart (410, 420, 430) for examining resolution of a fundus examining device, wherein the chart plates (310, 320, 330) are arranged at different positions so that the resolution test charts (410, 420, 430) are located at the focal distances of the measurement lights of the fundus examining device which incident to a central region and peripheral region of the inner rear surface of the model eye (10) through the lenses (100, 200).

### EFFECTS OF THE DISCLOSURE

According to the model eye of the present invention, it is possible to examine the resolution of a fundus examining device, specifically, both a resolution for central region and a resolution for peripheral region of a retina of a fundus examining device.

### BRIEF DESCRIPTION OF THE DRAWINGS

FIG. 1 is a drawing showing the structure of a model eye according to an embodiment of the present invention.
FIG. 2 is a drawing showing a side view and a top view of the chart unit of the model eye according to an embodiment of the present invention.
FIG. 3 is a drawing showing the structure of a model eye according to another embodiment of the present invention.
FIG. 4 is a drawing showing a side view and a top view of the chart unit of the model eye according to another embodiment of the present invention.
FIG. 5 is a drawing showing the chart unit of the model eye according to the present invention mounted on a holder.

### DETAILED DESCRIPTION OF THE PREFERRED EMBODIMENTS

Hereinafter, the present invention will be described in detail with reference to the accompanying drawings.

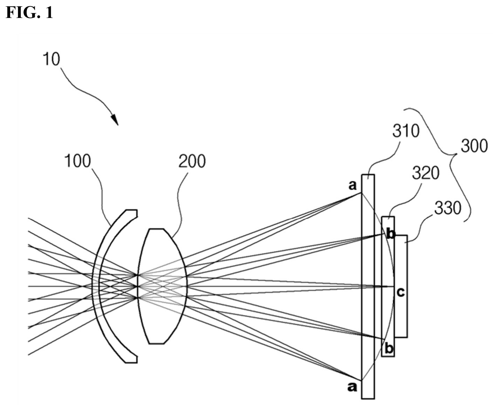

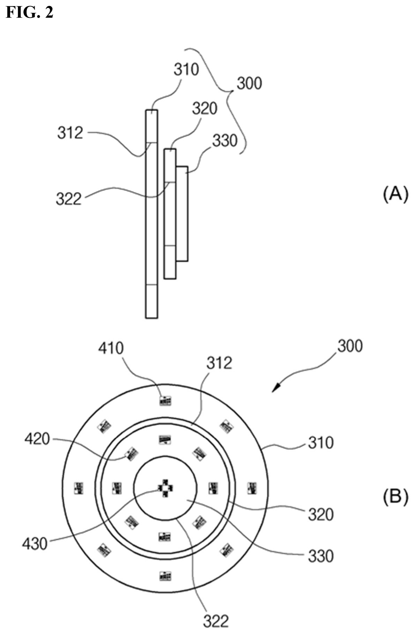

FIG. 1 is a drawing showing the structure of a model eye according to an embodiment of the present invention, and FIG. 2 is a drawing showing a side view and a top view of the chart unit of the model eye according to an embodiment of the present invention.

As shown in FIGS. 1 and 2, the model eye 10 according to an embodiment of the present invention includes one or more lenses 100, 200 and a chart unit 300.

The one or more lenses 100, 200 correspond to the cornea and/or crystalline lens of an eye and may include a cornea-simulating lens 100 corresponding to the cornea of an eye and a crystalline lens-simulating lens 200 corresponding to the crystalline lens of an eye. Preferably, both the cornea-simulating lens 100 and the crystalline lens-simulating lens 200 are included. Here, the correspondence of the cornea-simulating lens 100 and the crystalline lens-simulating lens 200 to the cornea and crystalline lens of an eye means that these lenses have characteristics, such as curvature and refractive index, of a standard cornea and crystalline lens, respectively. If necessary, the refractive power of the model eye 10 can be adjusted by adjusting the distance between the crystalline lens-simulating lens 200 and the cornea-simulating lens 100.

The chart unit 300 is located at the inner rear surface of the model eye 10, corresponding to the fundus of an eye, and includes two or more, for example, three chart plates 310, 320, 330 each having one or more resolution test charts 410, 420, 430 formed thereon for examining the resolution of a fundus examining device.

Here, the chart plates 310, 320, 330 are arranged at different positions so that the resolution test charts 410, 420, 430 are located at the focal distances of the measurement lights of a fundus examining device which incident to the central region and peripheral region of the inner rear surface of the model eye 10 through the lenses 100, 200.

For example, as shown in FIG. 1, the chart plates 310, 320, 330 may include a first chart plate 310, a second chart plate 320, and a third chart plate 330. The first chart plate 310, on which a first resolution test chart 410 is formed, is arranged at a position (a) in order to examine the resolution of the fundus examining device at the peripheral region of the retina. At this time, the measurement light of the fundus examining device for measuring the peripheral region of the retina is incident to the position (a) and forms a focus on the position (a) which corresponds to the peripheral region of the retina.

The third chart plate 330, on which a third resolution test chart 430 is formed, is arranged at a position (c) in order to examine the resolution of the fundus examining device at the central region of the retina. At this time, the measurement light of the fundus examining device for measuring the central region of the retina is incident to the position (c) and forms a focus on position (c) which corresponds to the central region of the retina.

The second chart plate 320, on which a second resolution test chart 420 is formed, is arranged at a position (b) which is a position between the first chart plate 310 and the third chart plate 330 in order to examine the resolution of the fundus examining device at an intermediate region between the central region and the peripheral region. At this time, the measurement light of the fundus examining device is incident to the position (b) and forms a focus on position (b) which corresponds to an intermediate region between the central and peripheral region of the retina.

The focus of the outermost measurement light passing through the crystalline lens-simulating lens 200 is formed on the first chart plate 310, the focus of the central measurement light passing through the crystalline lens-simulating lens 200 is formed on the third chart plate 330, and the focus of the intermediate measurement light between the outermost and central measurement light passing through the crystalline lens-simulating lens 200 is formed on the second chart plate 320.

As shown in FIG. 2, the first, second, and third chart plates 310, 320, 330 may have a ring shape with different diameters, be arranged in the form of concentric circles sharing the same center, and be located at different heights.

For example, when viewed from above (FIG. 2B), the second chart plate 320 is located inside a first through-hole 312 formed at the center of the first chart plate 310, and the third chart plate 330 is located inside a second through-hole 322 formed at the center of the second chart plate 320. When viewed from the side (FIG. 2A), the first chart plate 310, the second chart plate 320, and the third chart plate 330 are positioned parallel to each other at predetermined intervals.

Here, the diameter of the first chart plate 310 may be 15.6 to 19.6 mm, preferably 17.6 mm, the diameter of the second chart plate 320 may be 11 to 15 mm, preferably 13 mm, and the diameter of the third chart plate 330 may be 6 to 10 mm, preferably 8 mm. Additionally, the thickness of the first chart plate 310, the second chart plate 320, and the third chart plate 330 may each be 0.8 to 1.2 mm, for example, 1 mm.

The resolution test charts 410, 420, 430 may be formed on each of the chart plates 310, 320, 330 at predetermined intervals. Thus, when viewed from the side, the first resolution test chart 410, the second resolution test chart 420, and the third resolution test chart 430 may be arranged in parallel in a concave shape corresponding to the shape of the fundus of an eye.

The resolution test charts 410, 420, 430 are charts having a plurality of patterns of varying thickness and/or spacing. When images of the resolution test charts 410, 420, 430 are obtained using the fundus examining device, the resolutions of a fundus examining device can be determined or examined based on the degree to which the patterns of varying thickness and/or spacing are distinguished. For example, the more patterns of varying thickness and/or spacing are distinguished, the higher the resolution of the fundus examining device, and the fewer patterns are distinguished, the lower the resolution. As shown in FIG. 2, the first resolution test chart 410, the second resolution test chart 420, and the third resolution test chart 430 may have the same shape and size.

For example, the resolution test charts 410, 420, 430 may be the commonly used 1951 USAF resolution test chart.

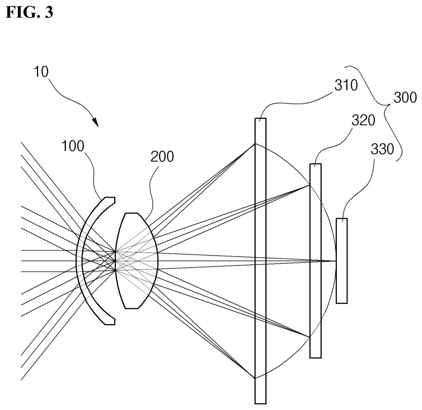

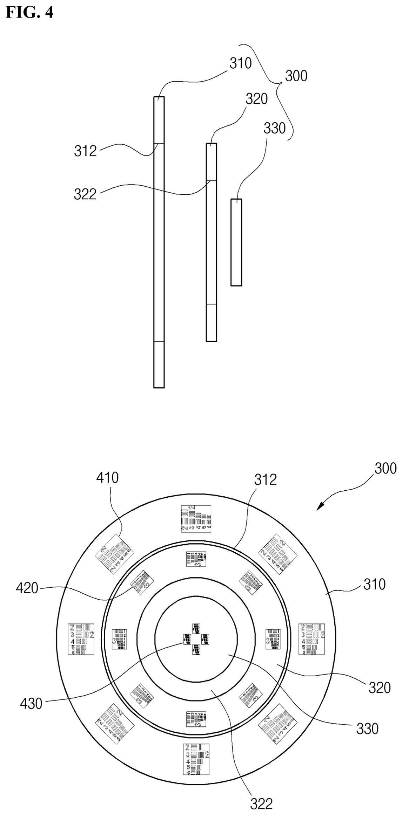

FIG. 3 is a drawing showing the structure of a model eye according to another embodiment of the present invention, and FIG. 4 is a drawing showing a side view and a top view of the chart unit of the model eye according to another embodiment of the present invention.

Compared to the model eye shown in FIGS. 1 and 2, the model eye shown in FIGS. 3 and 4 has a larger first chart plate 310 and/or a larger second chart plate 320. And the spacings between the first chart plate 310, the second chart plate 320, and the third chart plate 330 are adjusted to be suitable for examining the resolutions of a fundus examining device with a wider field of view (FOV).

For example, the field of view (FOV) of a fundus examining device that can be examined with the model eye shown in FIGS. 1 and 2 is 60°, while the field of view (FOV) of a fundus examining device that can be examined with the model eye shown in FIGS. 3 and 4 is 110°.

In the model eye shown in FIGS. 3 and 4, the first resolution test chart 410, the second resolution test chart 420, and the third resolution test chart 430 of the chart unit 300 have the same shape but decrease in size from the first resolution test chart 410 to the third resolution test chart 430.

The model eye according to the present invention may further include a holder 500 for supporting the chart unit 300.

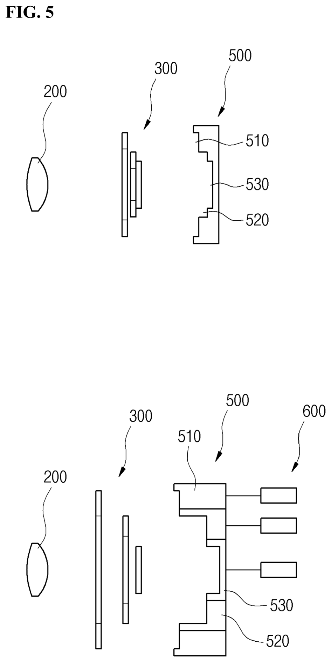

FIG. 5 is a drawing showing the chart unit 300 of the model eye according to the present invention mounted on the holder 500. As shown in FIG. 5, the first chart plate 310, the second chart plate 320, and the third chart plate 330 are mounted on a first holder 510, a second holder 520, and a third holder 530 of the holder 500, respectively. Specifically, the first chart plate 310 is mounted on the first holder 510, the second chart plate 320 is mounted on the second holder 520, and the third chart plate 330 is mounted on the third holder 530.

Additionally, the model eye according to the present invention may further include a position controller 600.

The position controller 600 adjusts the positions of the first holder 510, the second holder 520, and the third holder 530. Depending on the type of fundus examining device being examined or calibrated, the position controller 600 adjusts the positions of the first holder 510, the second holder 520, and the third holder 530 to adjust the positions of the first chart plate 310, the second chart plate 320, and the third chart plate 330, thereby enabling more effective examination of the resolutions of the fundus examining device.

Although the present invention has been described with reference to the accompanying drawings and exemplary embodiments, the present invention is not limited to the contents shown in the drawings or the embodiments described above. For clarity, reference numerals are included in the claims below, but the scope of the claims is not limited to the reference numerals or the contents shown in the drawings and should be interpreted to encompass all variations and equivalent configurations and functions of the exemplary embodiments.

## Claims

1. A model eye (10) comprising:
one or more lenses (100, 200) corresponding to a cornea and/or a crystalline lens of an eye; and
a chart unit (300) located at an inner rear surface of the model eye (10) corresponding to a fundus of an eye, and including two or more chart plates (310, 320, 330) each having one or more resolution test chart (410, 420, 430) for examining resolution of a fundus examining device,
wherein the chart plates (310, 320, 330) are arranged at different positions so that the resolution test charts (410, 420, 430) are located at the focal distances of the measurement lights of the fundus examining device which incident to a central region and peripheral region of the inner rear surface of the model eye (10) through the lenses (100, 200).

2. The model eye (10) according to claim 1, wherein the chart plates (310, 320, 330) include a first chart plate (310), a second chart plate (320), and a third chart plate (330),
wherein the first chart plate (310), on which a first resolution test chart (410) is formed, is arranged at a position (a) in order to examine the resolution of the fundus examining device at the peripheral region of the retina, wherein the measurement light of the fundus examining device for measuring the peripheral region of the retina is incident to the position (a) and forms a focus on the position (a) which corresponds to the peripheral region of the retina,
the third chart plate (330), on which a third resolution test chart (430) is formed, is arranged at a position (c) in order to examine the resolution of the fundus examining device at the central region of the retina, wherein the measurement light of the fundus examining device for measuring the central region of the retina is incident to the position (c) and forms a focus on position (c) which corresponds to the central region of the retina, and
the second chart plate (320), on which a second resolution test chart (420) is formed, is arranged at a position (b) which is a position between the first chart plate (310) and the third chart plate (330) in order to examine the resolution of the fundus examining device at an intermediate region between the central region and the peripheral region, wherein the measurement light of the fundus examining device is incident to the position (b) and forms a focus on position (b) which corresponds to the intermediate region between the central and peripheral region of the retina.

3. The model eye (10) according to claim 2, wherein the first chart plate (310), the second chart plate (320), and the third chart plate (330) have a ring shape with different diameters, are arranged in the form of concentric circles sharing the same center, and are located at different heights.

4. The model eye (10) according to claim 2, wherein the first resolution test chart (410), the second resolution test chart (420), and the third resolution test chart (430) are arranged in parallel in a concave shape corresponding to the shape of the fundus of an eye when viewed from the side.

5. The model eye (10) according to claim 1, wherein the resolution test charts (410, 420, 430) are charts having a plurality of patterns of varying thickness and/or spacing, and when images of the resolution test charts 410, 420, 430 are obtained using the fundus examining device, the resolutions of a fundus examining device can be determined based on the degree to which the patterns of varying thickness and/or spacing are distinguished.
